# EUROPÄISCHE PATENTSCHRIFT

(11) **EP 1 574 244 B1**
(45) Veröffentlichungstag und Bekanntmachung des Hinweises auf die Patenterteilung: **21.03.2012**
(21) Anmeldenummer: 05010479.3
(22) Anmeldetag: 19.11.1999
(51) Int. Cl.: B01D 63/02, A61M 1/18, B01D 65/00, B01D 46/24

(54) **Endkappe für eine Filtervorrichtung**
End cap for a filter device
Capuchon d'extrémité pour un filtre

(30) Priorität: 15.12.1998 DE 19857850
(43) Veröffentlichungstag der Anmeldung: 14.09.2005
(62) Teilanmeldung aus: 99123029.3
(73) Patentinhaber: Fresenius Medical Care Deutschland GmbH, 61352 Bad Homburg v.d.H. (DE)
(72) Erfinder: Heilmann, Klaus, 66606 St. Wendel (DE); Breith, Gerhard, 66606 St. Wendel (DE); Raiko, Igor, 66606 St. Wendel (DE); Sander, Roland, 66606 St. Wendel (DE); Fritzsche, Steffen, 66636 Tholey (DE)
(74) Vertreter: Laufhütte, Dieter

(56) Entgegenhaltungen:
- DE-A- 2 836 007
- DE-A- 3 435 883
- DE-A1- 19 716 646

## Beschreibung

Die vorliegende Erfindung betrifft eine Endkappe für eine Filtervorrichtung zum Stoffaustausch zwischen zwei durch eine Membran getrennte Medien sowie eine Filtervorrichtung, in der eine entsprechende Endkappe eingesetzt wird.

Derartige Filtervorrichtungen werden üblicherweise als Dialysatoren, Hämofilter oder Ultrafilter verwendet. Die Filtervorrichtungen bestehen im allgemeinen aus einem rohrabschnittförmigen Gehäuse, das in seinen Endbereichen mit Endkappen versehen ist. In dem Gehäuse ist üblicherweise ein als Membran dienendes Rohrfaserbündel derart angeordnet, daß eine Abdichtung zwischen dem durch die Faserhohlräume gebildeten ersten Strömungsraum und einem zweiten Strömungsraum, der die Außenseite der Membran umgibt, zuverlässig gegeben ist.

Bei der Gestaltung der Ein- und/oder Ausströmkammer, die mit dem ersten Strömungsraum, d.h. mit dem Hohlfaserbündel, in Verbindung steht, besteht ein Problem darin, die Flüssigkeit dergestalt zu verteilen, daß die einzelnen Fasern des Hohlfaserbündels in etwa gleichmäßig mit Flüssigkeit beaufschlagt werden und bei der Verteilung der Flüssigkeit Totzonen vermieden werden.

Es sind Dialysatoren bekannt, bei denen der Zulauf der mit dem Hohlfaserbündel in Verbindung stehenden Kammer axial angeordnet ist, wobei die Achse des Strömungskanals in etwa durch den Mittelpunkt des Hohlfaserbündels verläuft. Hierbei ergibt sich im allgemeinen der Nachteil, daß die Fasern stark unterschiedlich genutzt werden und gleichzeitig Bereiche auftreten, in denen die Strömungsgeschwindigkeit in etwa bei Null liegt (Totzonen). Derartige Totzonen sind nach Abschluß einer Dialysebehandlung die Bereiche, in denen Patientenblut zurückbleibt. Zusätzlich besteht ein großer Unterschied zwischen der Strömungsgeschwindigkeit im Zulauf und in den Fasern. Durch den dadurch verursachten Geschwindigkeitsgradienten wird das Blut einer Streßsituation ausgesetzt.

Aus der DE-OS 26 46 358 ist eine Filtervorrichtung bekannt, bei der die Endkappen einen tangentialen Zulauf aufweisen und das Blut in einem Kanal auf einer Kreisbahn über die Enden der Hohlfasern geführt wird. Das Blut strömt tangential über die Hohlfaserenden. Um eine möglichst gleichmäßige Flüssigkeitsverteilung zu erreichen, ist vorgesehen, daß nur die auf der überströmten Kreisbahn befindlichen Bereiche der Vergußmasse mit Hohlfasern versehen sind, während der restliche Kernbereich keine Fasern aufweist. Dadurch wird einerseits eine gleichmäßige Belastung der Fasern erreicht, andererseits ergibt sich aufgrund des Fehlens von Hohlfasern im Zentrum des Gehäuses insgesamt jedoch eine verhältnismäßig geringe Kapazität bzw. nicht optimale Ausnutzung der Filtervorrichtung. Um eine einheitliche Kreisflußgeschwindigkeit des Blutes im Kanal zu erzielen, weist dieser in einer Ausführungsform eine in Strömungsrichtung abnehmende Querschnittsfläche auf.

Aus der DE 197 16 646 A1 ist bereits ein Filter mit einem im Wesentlichen rohrförmigen Gehäuse bekannt, wobei die Verschlusskappen derart ausgebildet sind, dass sie sich von außen nach innen trompetenförmig erweitern und wobei in den Verschlusskappen jeweils ein Strömungsleitkörper angeordnet ist, so dass in den Verschlusskappen jeweils ein trichterförmiger Ringraum gebildet ist, durch den das zu reinigende Fluid in den Filter eingeführt bzw. aus dem Filter ausgeführt wird.

Die DE 34 35 883 A1 offenbart einen Hohlfaserdialysator, der im Zwischenraum zwischen der Endkappe und der Vergussschicht der Hohlfasern eine Strömungsleiteinrichtung aufweist, die sich quer durch den gesamten Zwischenraum erstreckt und am Außenumfang zwischen Abstandshaltern einen ringförmigen Schlitz aufweist, durch den die zugeführte Flüssigkeit strömen kann. Dabei wird die Flüssigkeit durch die Strömungsleiteinrichtung zunächst radial nach außen gelenkt und fließt nach dem Durchfließen der Strömungsleiteinrichtung radial nach innen zurück.

Die DE 28 36 007 A1 beschreibt eine Verteileranordnung für Hohlfaserdialysatoren, die aus zwei Raumabschnitten am Ende des Hohlfaserbündels besteht, wobei das Blut zuerst in den einen, ringförmigen Raumabschnitt eintritt und hier durch tangentiale Zufuhr eine primäre Kreisströmung erregt, wonach das Blut von hier in den anderen Abschnitt spiralförmig bei kombinierter Radial- und Kreisströmung übertritt, um von hier aus in die Hohlfasern einzutreten.

Es ist auch vorgeschlagen worden, den Zulauf parallel zur Ebene der Faserenden zu legen und im Endbereich des Zulaufs einen Richtungswechsel des Mediums um 90° vorzusehen. Bedingt durch den geringen verfügbaren Raum und Beschränkungen einer Fertigung durch Spritzgussverfahren ist im allgemeinen nur ein abrupter Richtungswechsel möglich, was zu erheblichen Belastungen des Blutes führen würde.

Es ist die Aufgabe der vorliegenden Erfindung, eine gattungsgemäße Endkappe für eine Filtervorrichtung dahingehend weiterzubilden, daß der Übergang des Blutes vom Zulauf in die an die Endkappe anschließende Filtervorrichtung und die dort aufgenommenen Fasern belastungsarm erfolgt und die Fasern in der Filtervorrichtung gleichmäßig beaufschlagt werden.

Diese Aufgabe wird erfindungsgemäß durch eine Endkappe für eine Filtervorrichtung gemäß Anspruch 1 gelöst. Vorteilhafte Ausgestaltungen der erfindungsgemäßen Endkappe sind Gegenstand der abhängigen Ansprüche 2 bis 15.

Ferner betrifft die vorliegende Erfindung eine Filtervorrichtung mit den Merkmalen des Anspruchs 16. Vorteilhafte Ausgestaltungen der erfindungsgemäßen Filtervorrichtung sind Gegenstand der abhängigen Ansprüche 17 bis 19.

Diese erfindungsgemäße Filtervorrichtung ist ausgestattet mit einem Kanal, der mit einem Zu- oder Ablauf der Endkappe und mit einer Ein- oder Ausströmkammer in Verbindung steht, wobei der Kanal das durch ihn geführte Fluid in eine erste Richtung leitet. Es sind hier Führungselemente vorhanden, die in dem sich um den Kanal erstreckenden Bereich angeordnet sind und dass den Kanal verlassende Fluid in einer definierten zweiten sich von der ersten unterscheidenden Richtung in der Ein- oder Ausströmkammer verteilt.

Erfindungsgemäß ergibt sich der Vorteil, dass die Blutströmung nur verhältnismäßig sanfte belastungsarme Änderungen der Geschwindigkeit in Richtung und Betrag erfährt. Die Strömungsgeschwindigkeit nähert sich bei der erfindungsgemäßen Vorrichtung beim Weg durch den gebogenen Kanal langsam den Wert der Geschwindigkeit in den Fasern an.

Zum anderen wird der Vorteil erreicht, daß das Blut auf dem Weg durch den Kanal durch die sich ändernde Querschnittsfläche annähernd radialsymmetrisch verteilt wird. Dies bedeutet, daß in jedem Winkelbereich in etwa der gleiche Betrag der Blutströmung den Kanal verläßt und radial nach außen fließt. Hieraus ergibt sich eine gleichmäßige Ausnutzung der einzelnen Fasern des Hohlfaserbündels und damit eine optimale Nutzung der Faserkapazität.

Im Gegensatz zu vorbekannten Lösungen kommt es bei der erfindungsgemäßen Anordnung zu keiner ausgeprägten Wirbelbildung, sondern zu einer vorwiegend gleichmäßigen radialsymmetrischen Verteilung der Flüssigkeit bzw. des Blutes.

Besonders vorteilhaft ist es, wenn die Hohlfasern in ihren Endbereichen in einer Vergußmasse aufgenommen sind, die sich parallel zu dem Kanal erstreckt. Die Vergußmasse ist üblicherweise scheibenförmig ausgeführt und die Hohlfaserenden sind in einer gleichmäßigen Verteilung in der Vergußmasse angeordnet.

Gemäß einer bevorzugten Ausgestaltung der vorliegenden Erfindung nimmt die Höhe und/oder Breite des Kanals in Strömungsrichtung ab. Besonders vorteilhaft ist es, wenn die Querschnittsfläche des Kanals linear mit dem Strömungsweg abnimmt. Durch eine derartige Ausgestaltung wird erreicht, daß pro Winkelbereich jeweils identische Mengen an Flüssigkeit bzw. Blut aus dem Kanal abgegeben werden, so daß eine annähernd radialsymmetrische Verteilung vorliegt.

Besonders vorteilhaft ist es, wenn ein sich radial erstreckender Zu- oder Ablauf vorgesehen ist, der in seinem einen Endbereich in den erfindungsgemäßen Kanal übergeht und in seinem anderen Endbereich die Mündung eines Anschlusses der Filtervorrichtung bildet.

Der Durchmesser des kreis- oder teilkreisförmigen Kanals kann vorteilhaft das 2- bis 4-fache, vorzugsweise das 2,5- bis 3,5-fache des Durchmessers des Zu- oder Ablaufes betragen.

In weiterer Ausgestaltung der vorliegenden Erfindung ist vorgesehen, daß der Durchmesser des kreis- oder teilkreisförmigen Kanals kleiner als 9/16, vorzugsweise kleiner als 3/8 des Durchmessers des Hohlfaserbündels ausgeführt ist.

Besonders vorteilhaft ist es, wenn der Kanal kreisförmig, d.h. umlaufend ausgeführt ist. Es ist somit nicht nur eine teilkreisförmige Ausführung des Kanals denkbar, sondern auch ein vollständiger Umlauf.

In weiterer Ausgestaltung der vorliegenden Erfindung ist vorgesehen, daß in den dem im wesentlichen kreis- oder teilkreisförmigen Kanal benachbarten Bereichen Führungselemente vorgesehen sind, mittels derer die Strömungsrichtung des den Kanal verlassenden Fluids beeinflußbar ist.

Die Führungselemente sind erfindungsgemäß als gekrümmte Rippen ausgeführt. Hierdurch wird es möglich, das Fluid, insbesondere das Blut, gleichmäßig und in definierter Art und Weise zu verteilen. Hierbei kann es zu der Ausbildung eines kleinen zentralen Wirbels kommen, der jedoch stets radialsymmetrisch ist.

Besonders vorteilhaft ist es, wenn die Rippen in Umfangsrichtung des Kanals äquidistant angeordnet sind. Darüber hinaus ist auch jede beliebige andere Anordnung von Rippen bzw. Führungselementen denkbar.

Das Hohlfaserbündel kann in einem rohrabschnittförmigen Gehäuse aufgenommen sein, wobei ein zweiter Strömungsraum durch den das Hohlfaserbündel umgebenden Gehäuseinnenraum gebildet wird und wobei die Ein- oder Ausströmkammer in einer mit dem Gehäuse stirnseitig in Verbindung stehenden Endkappe angeordnet ist. Es können in beiden Endbereichen des Gehäuses Endkappen vorgesehen sein.

Gemäß einer bevorzugten Ausführungsform der vorliegenden Erfindung erstreckt sich das Hohlfaserbündel über die gesamte Querschnittsfläche des Gehäuses. Ebenso ist es denkbar, daß nur Teilbereiche der Querschnittsfläche des Gehäuses bzw. der Vergußmasse mit Hohlfasern versehen sind.

Besonders vorteilhaft ist es, wenn jede der Endkappen zwei Anschlüsse aufweist, von denen einer mit dem ersten und einer mit dem zweiten Strömungsraum in Verbindung steht. Somit werden für jeden der Strömungsräume ein Zugang sowie ein Ablauf geschaffen, was entsprechend eine kontinuierliche Durchströmung beider Strömungsräume ermöglicht. Die Zuordnung als Zu- oder Ablauf ist nicht festgelegt, da die Anschlüsse je nach Bedarf vertauscht werden können.

In weiterer Ausgestaltung der vorliegenden Erfindung ist vorgesehen, daß die Anschlüsse radial angeordnet sind. Dies gilt entsprechend sowohl für die Blutseite, als auch für den Anschluß für die Dialyselösung, die üblicherweise durch den zweiten Strömungsraum geführt wird. Die radiale Anordnung der Anschlüsse kann zum einen strömungstechnische Vorteile haben. Zum anderen kann die Anordnung der Anschlüsse auch durch das zu verwendende Schlauchsystem bzw. durch die Halterung der Filtervorrichtung vorgegeben sein.

Gemäß einer bevorzugten Ausführungsform der vorliegenden Erfindung verlaufen die Mittellinien der Anschlüsse zueinander parallel. Es ist auch möglich, daß die Mittellinien nicht den Mittelpunkt der Endkappe durchlaufen.

Die Mündungen der Anschlüsse können in einer Ebene oder auch in zueinander parallelen Ebenen liegen. Die entsprechende Ausgestaltung ist im wesentlichen von der die Filtervorrichtung aufnehmenden Halterung abhängig, durch die ein rascher und zuverlässiger Wechsel der Filtervorrichtungen ermöglicht werden muß. In weiterer Ausgestaltung der vorliegenden Erfindung ist vorgesehen, daß die Anschlüsse in entgegengesetzte Richtungen weisen.

Die Endkappe ist erfindungsgemäß als Spritzgussteilausgeführt.

Besonders vorteilhaft ist es, wenn das Gehäuse sowie die Endkappen als Spritzgußteile ausgeführt sind. Hierdurch läßt sich eine verhältnismäßig einfache Herstellung gewährleisten, wobei zahireiche unterschiedliche Ausführungsformen denkbar sind.

Offenbart wird ferner eine Endkappe für eine Filtervorrichtung mit einer Ein- oder Ausströmkammer, die an einen im wesentlichen kreis- oder teilkreisförmigen Kanal angrenzt, der in Richtung zu den Enden der Hohlfasern eines in einem mit der Endkappe verbindbaren Filtervorrichtungsgehäuse angeordneten Hohlfaserbündels offen ausgeführt ist und mit einem Zu- oder Ablauf der Endkappe in Verbindung steht, der näherungsweise zentrisch zu dem Hohlfaserbündel angeordnet ist und eine in Strömungsrichtung abnehmende Querschnittsfläche aufweist und der einen Außendurchmesser aufweist, der unter dem Innendurchmesser des das Hohlfaserbündel aufnehmenden Bereichs der Endkappe liegt.

Weitere Einzelheiten und Vorteile der vorliegenden Erfindung werden anhand eines in der Zeichnung dargestellten Ausführungsbeispiels näher erläutert.

Es zeigen:
- Fig. 1:: eine Längsschnittansicht einer Endkappe mit Ein- oder Ausströmkammer mit teilkreisförmigem Kanal,
- Fig. 2:: eine teilweise Seitenansicht der Endkappe gemäß Fig. 1 gemäß Linie A-A,
- Fig. 3:: eine teilweise Seitenansicht der Endkappe gemäß Fig. 1 gemäß Linie B-B,
- Fig. 4:: eine Draufsicht auf die Endkappe gemäß Fig. 1,
- Fig. 5:: eine Detailansicht einer Schnittdarstellung gemäß Linie C-C in Fig. 4,
- Fig. 6:: eine Ansicht der Endkappe gemäß Fig. 1 von unten,
- Fig. 7:: eine schematische Darstellung der Größenverhältnisse von Zu- oder Ablauf, Kanal und Hohlfaserbündel
- Fig. 8:: eine Längsschnittansicht einer Endkappe mit Ein- oder Ausströmkammer mit kreisförmigen, d.h. umlaufenden Kanal,
- Fig. 9:: eine Draufsicht auf die Endkappe gemäß Fig. 8,
- Fig. 10:: eine Detailansicht einer Schnittdarstellung gemäß der Linie D-D in Fig. 9,
- Fig. 11:: eine Ansicht der Endkappe gemäß Fig. 8 von unten und
- Fig. 12:: eine Detailansicht des kreisförmigen Kanals sowie der benachbarten als Rippen ausgeführten Führungselemente gemäß Fig. 11

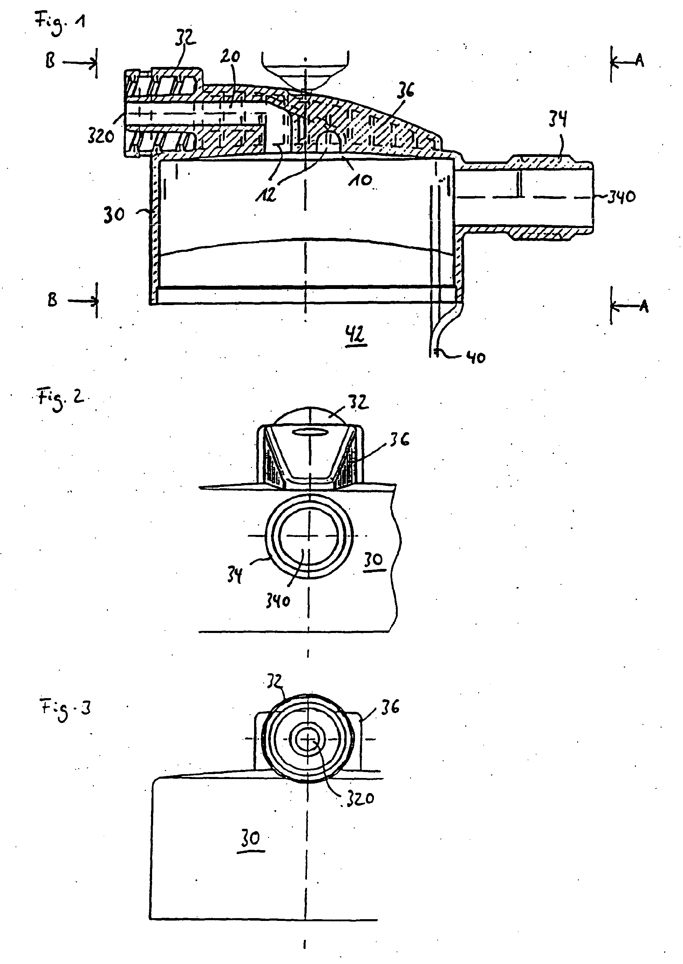

Fig. 1 zeigt in einer Längsschnittdarstellung die Endkappe 30, die flüssigkeitsdicht mit dem Gehäuse 40 in Verbindung steht. Die Endkappe 30 weist die Ein- oder Ausströmkammer 10 auf, die an den teilkreisförmigen Kanal 12 angrenzt. Das den Kanal 12 verlassende Fluid wird in die Ein- oder Ausströmkammer 10 geführt und annähernd radialsymmetrisch über die Enden der Hohlfasern eines nicht dargestellten Hohlfaserbündels verteilt.

Der Kanal 12 steht mit dem Zu- oder Ablauf 20 in Verbindung, der in seinem Endbereich die Mündung 320 des Anschlusses 32 der erfindungsgemäßen Filtervorrichtung bildet.

Die Endkappe 30 ist mit der Wulst 36 versehen, die sich radial erstreckt und in deren Endbereich der Anschluß 32 gebildet wird.

In dem in Fig. 1 rechts dargestellten Bereich der Endkappe 30 ist der Anschluß 34 vorgesehen, der die Mündung 340 aufweist, die mit dem zweiten Strömungsraum in Verbindung steht, der durch den die Fasern umgebenden Gehäuseinnenraum 42 gebildet wird.

Die Fig. 2 und 3 zeigen die seitlichen Ansichten der Endkappe 30 gemäß Fig. 1 in den durch die Linien A-A und B-B definierten Perspektiven. Hierbei wird deutlich, daß sowohl der Anschluß 32 als auch der Anschluß 34 radial angeordnet sind. Dies gilt ebenso für die Wulst 36, in deren Endbereich der Anschluß 32 angeordnet ist.

Die Mündungen 320 und 340 der Anschlußstutzen 32 und 34 liegen in zueinander parallelen Ebenen, weisen jedoch wie dies insbesondere aus Fig. 1 hervorgeht in entgegengesetzte Richtungen. Dies kann aus Gründen der Kompatibilität mit bereits vorhandenen Schlauchsystemen bzw. entsprechenden die Filter aufnehmenden Halterungen oder aufgrund von Handhabungsvorteilen erforderlich sein. Grundsätzlich ist es jedoch ebenso möglich, die Anschlüsse auf derselben Seite vorzusehen und die Mündungen in einer Ebene anzuordnen.

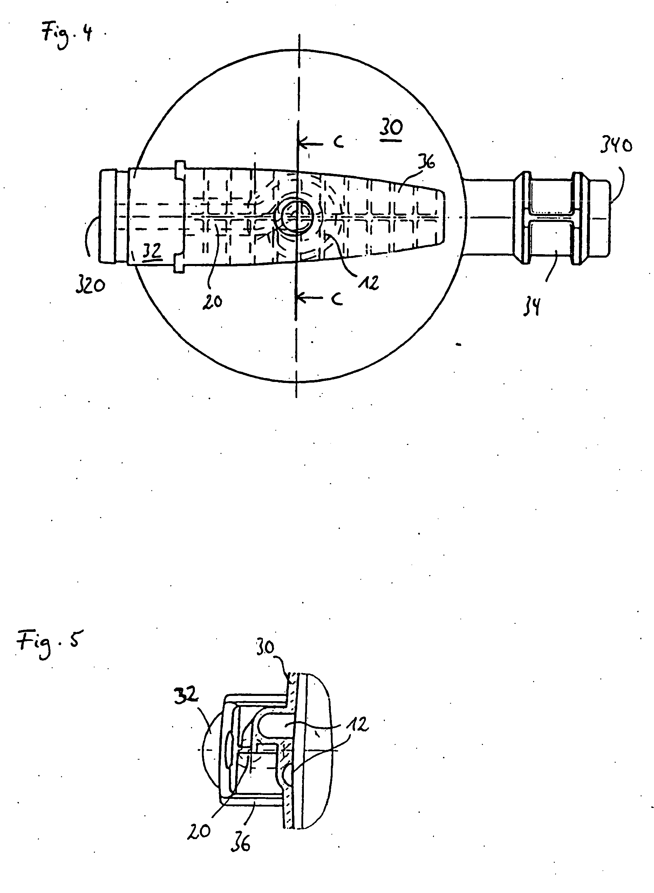

Fig. 4 zeigt in einer Draufsicht die Endkappe 30 und verdeutlicht durch die gestrichelte Linie die Anordnung und Ausgestaltung des Kanals 12 sowie der Zu- oder Ablaufleitung 20.

Fig. 4 zeigt, daß die durch den Anschluß 32 verlaufende Zu- oder Ablaufleitung 20 radial angeordnet ist. Die Zulaufleitung 20 geht in den Kanal 12 über, der erfindungsgemäß teilkreisförmig ausgeführt ist und sich in etwa zentrisch über dem Hohlfaserbündel bzw. der Vergußmasse erstreckt.

Der Kanal 12 weist eine in Strömungsrichtung abnehmende Höhe auf, was den Vorteil mit sich bringt, daß das durch den Kanal 12 geführte Fluid pro Winkelabschnitt in gleichen Anteilen, d.h. radialsymmetrisch, den Kanal 12 verläßt und in die Ein- oder Ausströmkammer 10 geführt wird und somit in gleichmäßiger Weise auf die Fasern des Hohlfaserbündels verteilt wird.

Gemäß dem vorliegenden Ausführungsbeispiel ist der Kanal 12 derart ausgeführt, daß dieser in seinem Endbereich die Höhe Null aufweist, d.h. in den Boden der Endkappe 30 übergeht.

Fig. 5 zeigt in einer Querschnittsdarstellung gemäß Linie C-C nach Fig. 4 die Anordnung und Ausgestaltung des Kanals 12. Hieraus wird deutlich, daß der in Fig. 5, rechts dargestellte Kanalquerschnitt gegenüber dem Zu- oder Ablauf 20 aufgrund der teilkreisförmigen Ausgestaltung versetzt ist und daß die Höhe des Kanals 12 in Strömungsrichtung abnimmt.

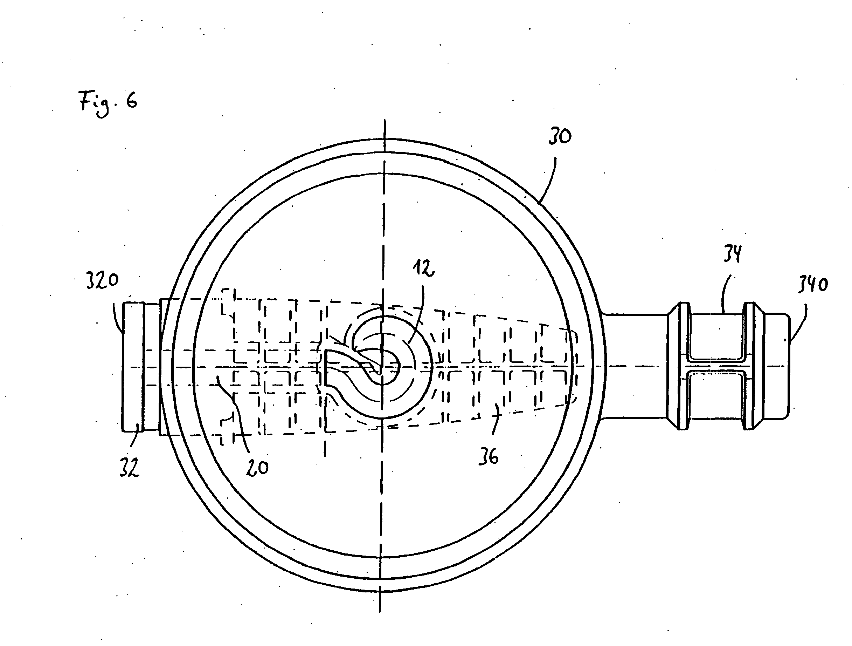

In Fig. 6 ist die Endkappe 30 gemäß Fig. 1 in einer Ansicht von unten dargestellt. Hierbei wird deutlich, daß der Kanal 12 eine teilkreisförmige Gestalt aufweist, und in seinem auslaufenden Endbereich in den Boden der Endkappe 30 übergeht. Fig. 6 zeigt ferner, daß der Mittelpunkt des durch den Kanal 12 gebildeten Teilkreises mit dem Mittelpunkt der Endkappe 30 und somit auch mit dem Mittelpunkt der Vergußmasse bzw. des dem Hohlfaserbündel übereinstimmt.

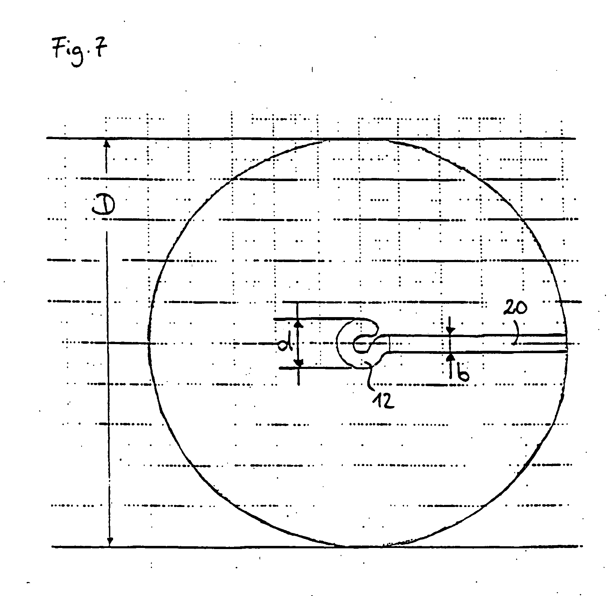

Fig. 7 zeigt eine schematische Darstellung der Größenverhältnisse des Zu- oder Ablaufs 20, des Kanals 12 und des durch den Kreis gekennzeichneten Hohlfaserbündels. Der Durchmesser (d) des kreis- oder teilkreisförmigen Kanals 12 beträgt gemäß dem vorliegenden Ausführungsbeispiel das 2- bis 4-fache, vorzugsweise das 2,5- bis 3,5-fache des Durchmessers (b) des Zu- oder Ablaufes 20. Der Durchmesser (d) des Kanals 12 ist kleiner als 9/16, vorzugsweise kleiner als 3/8 des Durchmessers (D) des Hohlfaserbündels ausgeführt.

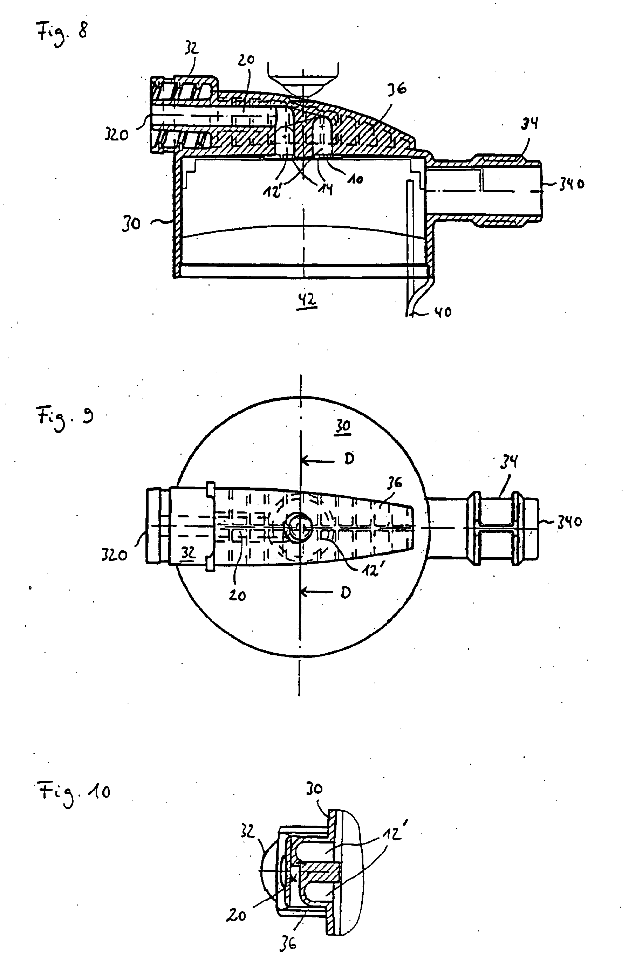

In Fig. 8 ist eine Längsschnittdarstellung einer weiteren Ausführungsform der erfindungsgemäßen Filtervorrichtung dargestellt. Die Endkappe 30 gemäß Fig. 8 weist einen kreisförmigen, d.h. umlaufenden Kanal 12' auf. Die Querschnittsfläche des Kanals 12' nimmt in Strömungsrichtung ab, was dadurch realisiert wird, daß dessen Höhe in Strömungsrichtung verringert wird, wie dies aus Fig. 8 hervorgeht.

In dem sich um den Kanal 12' erstreckenden Bereich sind die gekrümmten Rippen 14 angeordnet, mittels derer das den Kanal 12' verlassende Fluid in definierter Weise gleichmäßig in die Ein- oder Ausströmkammer 10 bzw. über das Hohlfaserbündel verteilt wird. Der Abstand der Rippen 14 zu der nicht näher dargestellten Vergußmasse des Filters ist vorteilhaft derart gering ausgeführt, daß eine fast vollständige Strömungsführung realisiert wird. Der Abstand liegt beispielsweise bei ca. 0,2 mm. Der Abstand des innerhalb des kreisförmigen Kanals 12' liegenden Stutzens der Endkappe 30 zu der Vergußmasse ist vorteilhaft etwas größer, beispielsweise 0,25 mm ausgeführt.

Fig. 9 zeigt eine Draufsicht auf die Endkappe 30 und verdeutlicht, daß der Kanal 12' gemäß dem Ausführungsbeispiel nach Fig. 8 umlaufend ausgeführt ist.

Aus Fig. 10 ist in einer Querschnittsdarstellung gemäß Linie D-D nach Fig. 9 die Anordnung und Ausgestaltung des Kanals 12' ersichtlich. Ein Vergleich mit Fig. 5 zeigt, daß bei der Ausführungsform gemäß Fig. 10 der unten dargestellte Abschnitt des Kanals eine größere Querschnittsfläche aufweist, die auch im weiteren Verlauf entsprechend der umlaufenden Ausführung des Kanals 12' nicht auf 0 zurückgeht.

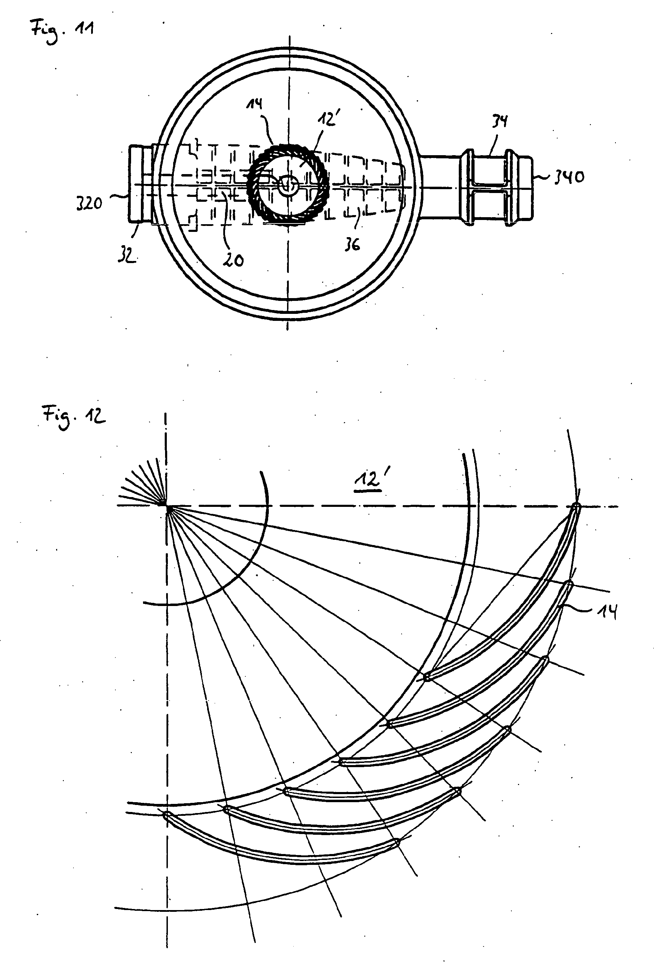

In Fig. 11 ist die Endkappe gemäß Fig. 8 in einer Ansicht von unten dargestellt. Hierbei wird nochmals deutlich, daß der Kanal 12' umlaufend ausgeführt ist. Ferner erkennt man, daß umlaufend um den Kanal 12' die Rippen 14 angeordnet sind, die nicht geradlinig radial nach außen, sondern gekrümmt angeordnet sind. Selbstverständlich ist neben der gezeigten Ausführung auch jede beliebige andere Anordnung und Ausführung der Rippen 14 denkbar. Die Rippen 14 dienen als Führungselemente für das den Kanal 12' verlassende Fluid. Die Rippen 14 sind beispielsweise ca. 1 mm oder einige 1/10 mm dick. Durch die Ausgestaltung und Anordnung derartiger Führungselemente wird es möglich, das den Kanal 12' verlassende Fluid in definierter Art und Weise und in definierter Richtung in der Ein- oder Ausströmkammer 10 bzw. über das Hohlfaserbündel zu verteilen.

Die Anordnung der Rippen 14 wird aus der Detailansicht gemäß Fig. 12 deutlich. Hierbei wird erkennbar, daß die Rippen 14 dem Kanal 12' unmittelbar benachbart sind. Die Rippen 14 sind äquidistant ausgeführt. Der Abstand der einzelnen Rippen nimmt in Strömungsrichtung ab, was zu einer entsprechenden Beschleunigung des durch die Rippen 14 geführten Fluids führt.

Die Anordnung von Führungselementen, insbesondere von Rippen 14, ist nicht nur bei der umlaufenden Ausführung des Kanals 12' denkbar, sondern selbstverständlich auch bei einer teilkreisförmigen Ausführung gemäß der Fig. 1-7.

Vorteilhafte Dimensionierungen des umlaufenden Kanals 12' gemäß der Fig. 8-12 ergeben sich entsprechend der Erläuterungen zu Fig. 7.

## Patentansprüche

1. Endkappe für eine Filtervorrichtung mit einem Kanal (12, 12'), der mit einem Zu- oder Ablauf (20) der Endkappe (30) und mit einer Ein- und Ausströmkammer (10) in Verbindung steht, wobei der Kanal das durch ihn geführte Fluid in eine erste Richtung leitet, und mit Führungselementen (14), die in dem sich um den Kanal (12, 12') erstreckenden Bereich angeordnet sind und das den Kanal verlassende Fluid in einer definierten zweiten sich von der ersten unterscheidenden Richtung in der Ein- oder Ausströmkammer (10) gleichmäßig verteilen,
wobei der Kanal (12, 12') an die Ein- oder Ausströmkammer (10) angrenzt, wobei der Kanal (12, 12') in Richtung zu den Enden der Hohlfasern eines in einem mit der Endkappe (30) verbindbaren Filtervorrichtungsgehäuse angeordneten Hohlfaserbündels offen ausgeführt ist und mit einem Zu- oder Ablauf (20) der Endkappe (30) in Verbindung steht,
wobei die Endkappe (30) als Spritzgussteil ausgeführt ist und
wobei die Führungselemente als gekrümmte Rippen (14) ausgeführt sind, die derart beschaffen sind, dass sie es ermöglichen, das Fluid gleichmäßig und in definierter Art und Weise zu verteilen, wobei ein kleiner zentraler Wirbel ausbildbar ist, der stets radialsymmetrisch ist.

2. Endkappe nach Anspruch 1, **dadurch gekennzeichnet, dass** der Kanal (12, 12') kreis- oder teilkreisförmig ist.

3. Endkappe nach Anspruch 1 oder 2, **dadurch gekennzeichnet, dass** der Kanal (12, 12') näherungsweise zentrisch zu dem Hohlfaserbündel angeordnet ist.

4. Endkappe nach einem der vorangehenden Ansprüche, **dadurch gekennzeichnet, dass** der Kanal (12, 12') eine in Strömungsrichtung abnehmende Querschnittsfläche aufweist.

5. Endkappe nach Anspruch 4, **dadurch gekennzeichnet, dass** die Querschnittsfläche linear mit dem Strömungsweg abnimmt.

6. Endkappe nach einem der vorhergehenden Ansprüche, **dadurch gekennzeichnet, dass** der Kanal (12, 12') einen Außendurchmesser aufweist, der unter dem Innendurchmesser des das Hohlfaserbündel aufnehmenden Bereichs liegt.

7. Endkappe nach einem der vorangehenden Ansprüche, **dadurch gekennzeichnet, dass** der Zu- oder Ablauf (20) sich radial erstreckt und in seinem einen Endbereich in den Kanal (12, 12') übergeht und in seinem anderen Endbereich die Mündung (320) eines Anschlusses (32) der Filtervorrichtung bildet.

8. Endkappe nach einem der vorangehenden Ansprüche, **dadurch gekennzeichnet, dass** der Durchmesser des kreis- oder teilkreisförmigen Kanals (12, 12') das 2 bis 4-fache, vorzugsweise das 2,5 bis 3,5-fache des Durchmessers des Zu- oder Ablaufes (20) beträgt.

9. Endkappe nach einem der vorhergehenden Ansprüche, **dadurch gekennzeichnet, dass** der Durchmesser des kreis- oder teilkreisförmigen Kanals (12, 12') kleiner als 9/16, vorzugsweise kleiner als 3/8 des Durchmessers des Hohlfaserbündels ist.

10. Endkappe nach einem der vorangehenden Ansprüche, **dadurch gekennzeichnet, dass** die Rippen (14) im Umfangsrichtung des Kanals (12, 12') äquidistant angeordnet sind.

11. Endkappe nach einem der vorangehenden Ansprüche, **dadurch gekennzeichnet, dass** sie zwei Anschlüsse (32, 34) aufweist, von denen einer mit dem ersten und einer mit dem zweiten Strömungsraum in Verbindung steht.

12. Endkappe nach Anspruch 11, **dadurch gekennzeichnet, dass** die Anschlüsse (32, 34) radial angeordnet sind.

13. Endkappe nach Anspruch 11 oder 12, **dadurch gekennzeichnet, dass** die Mittellinien der Anschlüsse (32, 34) zueinander parallel verlaufen.

14. Endkappe nach einem der Ansprüche 11-13, **dadurch gekennzeichnet, dass** die Mündungen (320, 340) der Anschlüsse (32, 34) in einer Ebene oder in zueinander parallelen Ebenen liegen.

15. Endkappe nach einem der Ansprüche 11-14, **dadurch gekennzeichnet, dass** die Anschlüsse (32, 34) in entgegengesetzte Richtungen weisen.

16. Filtervorrichtung mit einer Endkappe nach einem der vorangehenden Ansprüche, **dadurch gekennzeichnet, dass** das Hohlfaserbündel in einem rohrabschnittförmigen Gehäuse (40) aufgenommen ist, wobei ein zweiter Strömungsraum durch den das Hohlfaserbündel umgebenden Gehäuseinnenraum (42) gebildet wird und wobei die Ein- oder Ausströmkammer (10) in einer mit dem Gehäuse (40) stirnseitig in Verbindung stehenden Endkappe (30) angeordnet ist.

17. Filtervorrichtung nach Anspruch 16, **dadurch gekennzeichnet, dass** in beiden Endbereichen des Gehäuses (40) Endkappen (30) vorgesehen sind.

18. Filtervorrichtung nach Anspruch 16 oder 17, **dadurch gekennzeichnet, dass** sich das Hohlfaserbündel über die gesamte Querschnittsfläche des Gehäuses (40) erstreckt.

19. Filtervorrichtung nach einem der Ansprüche 16 bis 18, **dadurch gekennzeichnet, dass** das Gehäuse (40) sowie die Endkappen (30) als Spritzgußteile ausgeführt sind.

## Claims

1. An end cap for a filter apparatus having a channel (12, 12') which is in communication with a feed or discharge (20) of the end cap (30) and with an inflow and outflow chamber (10), wherein the channel conducts the fluid guided through it in a first direction, and having guide elements (14) which are arranged in the region extending around the channel (12, 12') and uniformly distribute the fluid exiting the channel in a defined second direction differing from the first direction in the inflow or outflow chamber (10),
wherein the channel (12, 12') is adjacent to the inflow or outflow chamber (10), with the channel (12, 12') being designed as open in the direction towards the ends of the hollow fibres of a hollow fibre bundle arranged in a filter apparatus housing connectable to the end cap (30) and being in communication with a feed or discharge (20) of the end cap (30),
wherein the end cap (30) is made as an injection moulded part, and
wherein the guide elements are made as curved ribs (14) which are constituted such that they allow the fluid to be distributed uniformly and in a defined manner, with a central small vortex being able to be formed which is always radially symmetrical.

2. An end cap in accordance with claim 1, **characterised in that** the channel (12, 12') is circular or in the form of a graduated circle.

3. An end cap in accordance with either of claims 1 or 2, **characterised in that** the channel (12, 12') is arranged approximately centrally to the hollow fibre bundle.

4. An end cap in accordance with one of the preceding claims, **characterised in that** the channel (12, 12') has a cross-section area which decreases in the flow direction.

5. An end cap in accordance with claim 4, **characterised in that** the cross-section area decreases in linear manner with the flow path.

6. An end cap in accordance with one of the preceding claims, **characterised in that** the channel (12, 12') has an outer diameter which is less than the inner diameter of the region receiving the hollow fibre bundle.

7. An end cap in accordance with one of the preceding claims, **characterised in that** the feed or discharge (20) extends radially, merges in its one end region into the channel (12, 12') and in its other end region forms the opening (320) of a connection (32) of the filter apparatus.

8. An end cap in accordance with one of the preceding claims, **characterised in that** the diameter of the channel (12, 12') which is circular or in the form of a graduated circle is 2 to 4 times, preferably 2.5 to 3.5 times the diameter of the feed or discharge (20).

9. An end cap in accordance with one of the preceding claims, **characterised in that** the diameter of the channel (12, 12') which is circular or in the form of a graduated circle is less than 9/16, preferably less than 3/8 of the diameter of the hollow fibre bundle.

10. An end cap in accordance with one of the preceding claims, **characterised in that** the ribs (14) are arranged equidistantly in the circumferential direction of the channel (12, 12').

11. An end cap in accordance with one of the preceding claims, **characterised in that** it has two connections (32, 34) of which one is connected to the first and one to the second flow chamber.

12. An end cap in accordance with claim 11, **characterised in that** the connections (32, 34) are arranged radially.

13. An end cap in accordance with either of claims 11 or 12, **characterised in that** the central lines of the connections (32, 34) run parallel to one another.

14. An end cap in accordance with one of the claims 11 - 13, **characterised in that** the openings (320, 340) of the connections (32, 34) lie in one plane or in planes which are parallel to one another.

15. An end cap in accordance with one of the claims 11 - 14, **characterised in that** the connections (32, 34) point in opposite directions.

16. A filter apparatus having an end cap in accordance with one of the preceding claims, **characterised in that** the hollow fibre bundle is received in a housing (40) in the form of a tubular section, wherein a second flow chamber is formed by the housing inner space (42) surrounding the hollow fibre bundle and wherein the inflow or outflow chamber (10) is arranged in the end cap (30) connected to the housing (40) on the face.

17. A filter apparatus in accordance with claim 16, **characterised in that** end caps (30) are provided in both end regions of the housing (40).

18. A filter apparatus in accordance with either of claims 16 or 17, **characterised in that** the hollow fibre bundle extends over the entire cross-section area of the housing (40).

19. A filter apparatus in accordance with one of the claims 16 to 18, **characterised in that** the housing (40) and the end caps (30) are made as injection moulded parts.

## Revendications

1. Capuchon d'extrémité destiné à un dispositif de filtrage avec un canal (12, 12'), qui est relié à une arrivée ou sortie (20) du capuchon d'extrémité (30) et se trouve en relation avec une chambre d'admission ou d'échappement (10), le canal circulaire guidant le liquide conduit par lui dans une première direction, et avec des éléments d'entraînement (14), qui sont disposés dans la zone s'étendant autour du canal (12, 12') et répartissent uniformément le fluide quittant le canal dans la chambre d'admission ou d'échappement (10) dans une deuxième direction définie se distinguant de la première,
le canal (12, 12') étant limitrophe de la chambre d'admission ou d'échappement (10), le canal (12, 12') étant construit de manière ouverte dans la direction vers les extrémités des fibres creuses d'un faisceau de fibres creuses disposé dans un carter de dispositif de filtrage reliable avec le capuchon d'extrémité (30) et se trouvant en relation avec une arrivée ou sortie (20) du capuchon d'extrémité (30), le capuchon d'extrémité (30) étant construit comme pièce moulée par injection et les éléments d'entraînement étant construits comme membrures arquées (14), qui sont constituées de telle manière qu'elles permettent de répartir le fluide uniformément et d'une manière définie, un petit tourbillon central étant constituable, qui est toujours à symétrie radiale.

2. Capuchon d'extrémité selon la revendication 1, **caractérisé en ce que** le canal (12, 12') est circulaire ou à section circulaire.

3. Capuchon d'extrémité selon la revendication 1 ou 2, **caractérisé en ce que** le canal (12, 12') est disposé de manière approximativement centrée par rapport au faisceau de fibres creuses.

4. Capuchon d'extrémité selon une quelconque des revendications précédentes, **caractérisé en ce que** le canal (12, 12') présente une superficie de la section décroissante dans la direction de l'écoulement.

5. Capuchon d'extrémité selon la revendication 4, **caractérisé en ce que** la superficie de la section décroit linéairement avec la trajectoire d'écoulement.

6. Capuchon d'extrémité selon une quelconque des revendications précédentes, **caractérisé en ce que** le canal (12, 12') présente un diamètre extérieur, qui se trouve en-dessous du diamètre intérieur de la zone supportant le faisceau de fibres creuses.

7. Capuchon d'extrémité selon une quelconque des revendications précédentes, **caractérisé en ce que** l'arrivée ou sortie (20) s'étend radialement et se transforme dans l'une de ses zones terminales en canal (12, 12') et constitue dans l'autre de ses zones terminales l'embouchure (320) d'un raccord (32) du dispositif de filtrage.

8. Capuchon d'extrémité selon une quelconque des revendications précédentes, **caractérisé en ce que** le diamètre du canal circulaire ou à section circulaire (12, 12') s'élève de 2 à 4 fois, de préférence de 2,5 à 3,5 fois le diamètre de l'arrivée ou la sortie (20).

9. Capuchon d'extrémité selon une quelconque des revendications précédentes, **caractérisé en ce que** le diamètre du canal circulaire ou à section circulaire (12, 12') est inférieur à 9/16, de préférence inférieur à 3/8 du diamètre du faisceau de fibres creuses.

10. Capuchon d'extrémité selon une quelconque des revendications précédentes, **caractérisé en ce que** les membrures (14) sont disposées de manière équidistante dans la direction circonférentielle du canal (12, 12').

11. Capuchon d'extrémité selon une quelconque des revendications précédentes, **caractérisé en ce qu'**il présente deux raccords (32, 34), dont l'un est en relation avec la première et l'un avec la deuxième chambre d'écoulement.

12. Capuchon d'extrémité selon la revendication 11, **caractérisé en ce que** les raccords (32, 34) sont disposés radialement.

13. Capuchon d'extrémité selon la revendication 11 ou 12, **caractérisé en ce que** les lignes médianes des raccords (32, 34) se déroulent parallèlement l'une à l'autre.

14. Capuchon d'extrémité selon une quelconque des revendications 11 à 13, **caractérisé en ce que** les embouchures (320, 340) des raccords (32, 34) se trouvent sur un plan ou sur des plans parallèles l'un à l'autre.

15. Capuchon d'extrémité selon une quelconque des revendications 11 à 14, **caractérisé en ce que** les raccords (32, 34) sont orientés dans des directions opposées.

16. Dispositif de filtrage avec un capuchon d'extrémité selon une quelconque des revendications précédentes, **caractérisé en ce que** le faisceau de fibres creuses est supporté dans un carter en forme de section tubulaire (40), une deuxième chambre d'écoulement étant constituée par l'espace intérieur de carter (42) entourant le faisceau de fibres creuses et la chambre d'admission ou d'échappement (10) étant disposée dans un capuchon d'extrémité (30) se trouvant frontalement en relation avec le carter (40).

17. Dispositif de filtrage selon la revendication 16, **caractérisé en ce que**, dans les deux zones terminales du carter (40), des capuchons d'extrémité (30) sont prévus.

18. Dispositif de filtrage selon la revendication 16 ou 17, **caractérisé en ce que** le faisceau de fibres creuses s'étend sur la superficie complète de la section du carter (40).

19. Dispositif de filtrage selon une quelconque des revendications 16 à 18, **caractérisé en ce que** le carter (40) ainsi que les capuchons d'extrémité (30) sont construits comme pièces moulées par injection.
